# EUROPÄISCHE PATENTSCHRIFT

(11) **EP 0 680 727 B1**
(45) Veröffentlichungstag und Bekanntmachung des Hinweises auf die Patenterteilung: **18.06.2003**
(21) Anmeldenummer: 95106046.6
(22) Anmeldetag: 22.04.1995
(51) Int. Cl.: A61B 5/00

(54) **Analysesystem zur Überwachung der Konzentration eines Analyten im Blut eines Patienten**
Analysing system for monitoring the concentration of an analyte in the blood of a patient
Sytème d'analyse pour surveiller la concentration d'un analyte dans le sang d'un patient

(30) Priorität: 05.05.1994 DE 4415896
(43) Veröffentlichungstag der Anmeldung: 08.11.1995
(73) Patentinhaber: Roche Diagnostics GmbH, 68298 Mannheim (DE)
(72) Erfinder: Böcker, Dirk, Dr.Dr., D-69115 Heidelberg (DE); Haar, Hans-Peter, Dr., D-69168 Wiesloch (DE); Blasberg, Peter, D-69469 Weinheim (DE); Kotulla, Reinhard, Dr., D-67245 Lambsheim (DE)
(74) Vertreter: Pfeifer, Hans-Peter, Dr.

(56) Entgegenhaltungen:
- EP-A- 0 492 326
- WO-A-92/00513
- WO-A-92/22804
- US-A- 4 140 963

## Beschreibung

Die Erfindung betrifft ein Analysesystem zur Überwachung der Konzentration eines Analyten im Blut eines Patienten.

Vielfach ist es erforderlich, die Konzentration eines Analyten im Blut regelmäßig zu überwachen. Dies gilt insbesondere bei Krankheiten, bei denen eine regelmäßige medikamentöse Behandlung in Abhängigkeit von der jeweiligen Analyt-Konzentration erforderlich ist. Das wichtigste Beispiel ist der Diabetes-Mellitus (Zuckerkrankheit). Patienten, die an dieser Krankheit leiden, sollten ihren Blutzuckerspiegel laufend überwachen, um ihre Insulininjektionen jeweils dem aktuellen Bedarf anpassen zu können und dadurch ihre Blutzuckerwerte (d.h. die Glucosekonzentration im Blut) innerhalb bestimmter Grenzen zu halten. Sowohl ein Überschreiten dieser Grenzen (Hyperglykämie), als auch ein Unterschreiten dieser Grenzen (Hypoglykämie) soll mit größtmöglicher Sicherheit vermieden werden, um sowohl kritische Akutzustände, als auch schwerwiegende Langzeitschäden (wie beispielsweise Verlust des Augenlichts) zu vermeiden.

Die vorliegende Erfindung richtet sich insbesondere auf die Überwachung der Glucosekonzentration im Blut, ist jedoch auch für andere Analyten verwendbar. Soweit nachfolgend beispielhaft auf die Glucosebestimmung Bezug genommen wird, soll dies nicht als Beschränkung der allgemeinen Anwendbarkeit der Erfindung verstanden werden.

Zur Überwachung der Glucosekonzentration im Blut sind Analysesysteme gebräuchlich, die aus Analyseelementen (solid state analysis elements), welche auch als Testträger (test carrier) bezeichnet werden, und einem Auswertegerät bestehen. Die Analyseelemente und das Auswertegerät sind in der Regel speziell aufeinander abgestimmt und werden vom gleichen Hersteller als System angeboten.

Die Analyseelemente enthalten Reagenzien. Wenn man sie mit der Probe kontaktiert, führt die Reaktion des in der Probe enthaltenen Analyten mit den Reagenzien zu einer physikalisch meßbaren Veränderung des Analyseelementes, die mit der Konzentration des Analyten korreliert. Das Auswertegerät enthält eine Meßeinrichtung zum Messen der Veränderung und eine elektronische Schaltung zur Bestimmung der Konzentration des Analyten aus dem bei der Messung der Veränderung gemessenen Meßwert. Für die Auswerteelektronik werden bei modernen Geräten Mikroprozessoren verwendet, die eine softwaregesteuerte digitale Verarbeitung der Meßwerte zu der Konzentration des Analyten entsprechenden elektrischen Signalen ermöglichen. Diese Analysedaten werden in der Regel auf einem alphanumerischen Display in Konzentrationseinheiten angezeigt. Als Analysedaten im Sinne der Erfindung sind jedoch auch solche elektrische Signale zu verstehen, die das Analyseergebnis in anderer Weise repräsentieren, beispielsweise Signale zur Ansteuerung stufenweiser Anzeigen von Informationen über die Konzentration des Analyten, wie "Idealbereich", "oberer Normbereich", "oberer Gefahrenbereich", etc.

Es sind unterschiedliche Typen von Analyseelementen bekannt, die hinsichtlich der Reaktionsprinzipien und der mit der Konzentration korrelierenden meßbaren Veränderung unterschiedliche chemisch-physikalische Prinzipien verwenden. Gebräuchlich sind vor allem photometrische und elektrochemische Analysesysteme.

Bei photometrischen Analysesystemen enthalten die Analyseelemente ein Reagenzsystem, dessen Reaktion mit dem Analyten zu einer photometrisch nachweisbaren Veränderung (einem Farbumschlag) führt. Die Reagenzien befinden sich dabei üblicherweise in einer Matrix aus porösem Kunststoff oder Papier, die ein Testfeld des Analyseelementes bildet und deren Farbe sich in Abhängigkeit von der Konzentration ändert. Diese Farbänderung läßt sich quantitativ mit Hilfe der Reflexionsphotometrie bestimmen.

Elektrochemische Analyseelemente enthalten ein elektrochemisches Reagenzsystem, dessen Reaktion mit dem Analyten die zwischen zwei Polen des Analyseelementes anliegende elektrische Spannung und/oder die zwischen zwei Polen des Analyseelementes bei definierter Spannung fließende Stromstärke beeinflußt. In diesem Fall ist also die Spannung oder Stromstärke die physikalisch meßbare Meßgröße, die mit einer entsprechenden in dem Auswertegerät integrierten Spannungs- oder Strommeßeinrichtung bestimmt und deren mit der Konzentration des Analyten korrelierende Änderung -wiederum bevorzugt mit einer Mikroprozessor-Auswerteelektronik- in die Analysedaten (Konzentration des Analyten) umgerechnet wird.

Analysesysteme, die mit Analyseelementen arbeiten ("Element-Analysesysteme"), haben einen hohen Grad von Genauigkeit erreicht und sind so einfach zu handhaben, daß sie von dem Patienten selbst zur laufenden Überwachung seiner Blutglucose-Konzentration verwendet werden können ("home monitoring"). Sie haben jedoch den wesentlichen Nachteil, daß für jede einzelne Analyse ein Tropfen Blut gewonnen werden muß, der mit dem Analyseelement kontaktiert wird. Dies geschieht in der Regel durch einen Stich in den Finger, d.h. es ist für jede Analyse eine schmerzhafte und mit einem gewissen Infektionsrisiko verbundene Verletzung der Haut erforderlich. Man spricht deshalb auch von invasiven Analyseverfahren.

Um eine kontinuierliche Überwachung der Konzentration eines Analyten im Blut eines Patienten mit guter Genauigkeit und einer verminderten Zahl von invasiven Eingriffen zur Probengewinnung zu ermöglichen, wird ein Analysesystem gemäß Anspruch 1 vorgeschlagen.

Die Erfindung verknüpft auf neuartige Weise ein für die Blutanalyse gebräuchliches Element-Analysesystem der vorstehend erläuterten Art mit einem nicht invasiv arbeitenden Analysesystem, das eine am Körper des Patienten tragbare Sensoreinheit zur reagenzienfreien unmittelbaren Messung eines mit der Konzentration des Analyten korellierenden Parameters am Körper des Patienten und eine Sensor-Auswerteelektronik zur Ermittlung von Sensor-Analysedaten aus den Sensor-Meßdaten des gemessenen Parameters aufweist. Die beiden Teil-Systeme bilden ein integriertes Gesamtsystem, das es ermöglicht, Analysewerte, insbesondere Glycosewerte praktisch kontinuierlich zuverlässig zu bestimmen. Kalibrationen mittels einer Element-Analyse können jederzeit durchgeführt werden, so daß das tägliche Leben des Patienten minimal beeinträchtigt wird. Da die Kalibration nach Durchführung einer Element-Analyse vollständig automatisch abläuft, werden Fehler durch Fehlbedienungen vermieden.

Sensor-Analysesysteme zur reagenzienfreien unmittelbaren Bestimmung von Analyten im Blut sind bereits in unterschiedlichen Ausführungsformen beschrieben worden. Für einige Analyten (insbesondere die Blut-Oxygenierungswerte sowie Blutgaskonzentrationen) haben sie auch praktische Bedeutung erlangt. Für eine Vielzahl anderer Analyten, insbesondere Glucose, haben sie sich bisher in der Praxis nicht ausreichend bewährt.

Ein Überblick über nichtinvasive Methoden zur Bestimmung von Glucose wird in dem Artikel von J.D. Kruse-Jarres "Physicochemical Determinations of Glucose in vivo", J. Clin. Chem. Clin. Biochem. 26 (1988), 201-208 gegeben.

Die Erfindung richtet sich insbesondere auf Systeme, bei denen die Wechselwirkung von in das Gewebe eines lebenden Menschen (vorzugsweise das Hautgewebe) eingestrahltem Licht mit dem Gewebe zur analytischen Bestimmung des darin vorhandenen Analyten verwendet wird. Dabei wird davon ausgegangen, daß die Konzentration des Analyten in dem (durchbluteten) Gewebe in einem für praktische Zwecke ausreichendem Maße mit der entsprechenden Konzentration im Blut korreliert. Bei diesen Systemen weist die Sensoreinheit einen Lichtsender auf, von dem Licht in das Gewebe eingestrahlt wird. Weiter ist ein Lichtempfänger vorhanden, durch den nach Wechselwirkung mit dem Gewebe aus dem Körperteil austretendes Licht detektiert wird, um eine durch die Wechselwirkung mit dem Gewebe veränderliche meßbare physikalische Eigenschaft des Lichts zu bestimmen. Diese meßbare physikalische Eigenschaft bildet bei solchen Verfahren einen mit der Konzentration des Analyten korrelierenden Parameter.

Den meisten bisher bekannt gewordenen Verfahren dieser Art liegen die Prinzipien der Spektralanalyse zugrunde. Dabei wird die charakteristische Absorption des Analyten (welche auf Schwingungs- und Rotationszustände des Analyt-Moleküls oder von Teilen desselben zurückgeht) bestimmt, indem man die Abhängigkeit der optischen Absorption von der Licht-Wellenlänge untersucht. In der Praxis wird meist Licht unterschiedlicher Wellenlängen von einer schmalbandig emittierenden Lichtquelle eingestrahlt und das dabei von dem Lichtempfänger empfangene Licht gemessen. Alternativ kann auch mit einer breitbandigen Lichtquelle eingestrahlt und auf der Detektionsseite eine wellenlängenselektive Messung durchgeführt werden. Die Absorptionsbanden der in Rede stehenden Moleküle (insbesondere der Glucose) liegen weit im infraroten Bereich des Lichtes. Da dort jedoch das in dem Gewebe enthaltene Wasser sehr stark absorbiert, werden von den meisten Autoren Meßwellenlängen im Bereich des nahen Infrarot-Lichtes vorgeschlagen, mit denen Oberwellen der Molekül-Schwingungs- und Rotationszustände erfaßt werden können. Solche Systeme werden beispielsweise beschrieben in der EP-A-0 160 768, der WO 93/00856 und dem US-Patent 5,028,787.

Ein Sensor-Analysesystem, bei dem NIR-Spektraldaten ausgewertet werden, ist beispielsweise auch in der WO 92/00513 beschrieben. Da Sensor-Analysesysteme in aller Regel keine Absolutmessung ermöglichen, ist eine Kalibration erforderlich, die bei dem in diesem Dokument beschriebenen System mittels eines mit Teststreifen arbeitenden konventionellen Analysesystems erfolgt. Es wird ein besonderes Kalibrationsverfahren beschrieben, bei dem eine größere Anzahl von invasiven Messungen in regelmäßigen Abständen von beispielsweise 15 Minuten durchgeführt werden. Die dabei gewonnenen invasiven Analysedaten werden mittels einer Regressionanalyse mit Sensor-Analysedaten verglichen, die im gleichen Zeitraum gewonnen wurden, wobei die Zahl der Sensor-Messungen sehr viel größer als die Zahl der invasiven Messungen ist. Außerdem wird zu Beginn des Kalibrationszeitraumes dem Patienten ein Glucosetrank verabreicht, um gezielt einen Anstieg seines Blutglucosewertes innerhalb des Kalibrationszeitraumes zu erreichen. Durch dieses aufwendige Verfahren soll erreicht werden, daß nur eine einzige Kalibration des nichtinvasiven Sensor-Analysesystems erforderlich ist.

Besonders bevorzugt wird bei der Erfindung ein Sensorsystem eingesetzt, bei dem ein Parameter des Lichtes bestimmt wird, der von dem Brechungsindex in dem Gewebe beeinflußt wird. Eine wichtige Grundlage dieser Verfahrensweisen ist die Erkenntnis, daß die mit Änderungen der Glucosekonzentration verbundene Änderung des Brechungsindex der Flüssigkeit in dem Gewebe als mit der Glucosekonzentration korrelierender Parameter verwendet werden kann.

Zur meßtechnischen Realisierung ist insbesondere vorgeschlagen worden, ein von der Vielfachstreuung des Lichts durch Streuzentren in dem Gewebe beeinflußtes Signal zu bestimmen. Diese Verfahrensweise wird in der internationalen Patentanmeldung WO-A-9410901 (PCT/DE 93/01058) beschrieben. Die Vielfachstreuung führt unter den in dieser Literaturstelle beschriebenen meßtechnischen Bedingungen zu einer Verstärkung des mit der Änderung des Brechungsindex verbundenen Effektes, die als verhältnismäßig starke und deswegen gut meßbare Signaländerung bestimmt werden kann.

Nähere Einzelheiten werden in der Literaturstelle erörtert, auf die hier vollinhaltlich Bezug genommen wird.

In der deutschen Patentanmeldung 43 37 570 ist die Analyse von Glucose auf Basis der Bestimmung eines der Laufzeit des Lichts in dem Gewebe entsprechenden Licht-Parameters beschrieben. Dieser "Laufzeit-Parameter" kann unmittelbar die Laufzeit eines extrem kurzen Lichtimpulses sein. Meßtechnisch erheblich weniger aufwendig ist es, stattdessen die Phasenverschiebung des Lichts innerhalb des Gewebes als Laufzeit-Parameter zu ermitteln, der mit der Glucosekonzentration in dem Gewebe korreliert. Auch zu dieser Verfahrensweise sind weitere Einzelheiten in der genannten Patentanmeldung beschrieben, auf die hier vollinhaltlich Bezug genommen wird.

Schließlich ist in der gleichzeitig eingereichten deutschen Patentanmeldung "Verfahren und Vorrichtung zur Analyse von Glucose in einer biologischen Probe" (Aktenzeichen: 44 15 728) beschrieben, Änderungen des Brechungsindex in dem Gewebe mit Hilfe der Niederkohärenz-Interferometrie zu bestimmen. Dies kann entweder unmittelbar durch Bestimmung der optischen Weglänge des Lichts in dem Gewebe oder indirekt in der Weise geschehen, daß man den Streukoeffizienten des Lichts in dem Gewebe bestimmt. Der Streukoeffizient seinerseits wird entscheidend durch die Relation zwischen dem Brechungsindex der Flüssigkeit und dem Brechungsindex der in dem Gewebe enthaltenen Streuzentren (beispielsweise Zellen) beeinflußt. Auch auf diese Anmeldung wird hier vollinhaltlich Bezug genommen.

Gemäß der Erfindung wird ein solches nichtinvasives Sensor-Analysesystem mit einem invasiven mit Reagenzien arbeitenden Analyseelement-Analysesystem kombiniert. Dabei besteht das Sensor-Analysesystem aus einer am Körper des Patienten tragbaren, mobilen, batteriebetriebenen Sensoreinheit und einem Auswertegerät, welches in dem Sinn stationär ist, daß es nicht am Körper des Patienten getragen, sondern an geeigneter Stelle beispielsweise in der Wohnung des Patienten positioniert wird. Die Zentraleinheit ist vorzugsweise jedoch so klein und so leicht, daß der Patient sie leicht mitnehmen kann, wenn er für längere Zeit (beispielsweise für mehrere Tage) seine Wohnung verläßt. Das Auswertegerät und die Zentraleinheit stehen in einer drahtlosen Datenübertragungs- Verbindung zueinander. Diese kann auf unterschiedliche Weise realisiert sein, beispielsweise mit IR-Licht, Hochfrequenz-Radiowellen oder auch Ultraschall.

Die am Körper des Patienten tragbare Sensoreinheit und die stationäre Zentraleinheit erfüllen gemeinsam die Funktionen des Sensor-Analysesystems, wobei die Aufteilung der Systemfunktionen auf die beiden Einheiten in unterschiedlicher Weise realisiert sein kann. Grundsätzlich kann eine Sensoreinheit ohne eigene Intelligenz realisiert sein, wobei sie lediglich die Sensor-Meßwerte bestimmt und drahtlos an die Zentraleinheit weitergibt. Vorzugsweise ist jedoch die Sensoreinheit mit eigener Intelligenz ausgestattet, d.h. sie besitzt ein Mikroprozessor-Datenverarbeitungssystem als Auswertemittel, um aus den Meßwerten des mindestens einen Sensors der Sensoreinheit (der Analyt-Konzentration entsprechende) Analysedaten zu ermitteln. Dadurch besteht die Möglichkeit, die Sensoreinheit mit einer eigenen Anzeige von Analyt-Konzentrationsdaten zu versehen, welche beispielsweise als einfache Warnanzeige eine akustische oder optische Warnung abgibt, wenn bestimmte Grenzwerte der Glucosekonzentration unter- oder überschritten werden. In diesem Fall ist es weiterhin vorteilhaft, wenn die Verbindung zwischen der Sensoreinheit und der Zentraleinheit interaktiv ist, d.h. es werden nicht nur Analyseelement-Analysedaten von der Sensoreinheit an die Zentraleinheit übermittelt, sondern auch umgekehrt Daten von der Zentraleinheit für die Sensoreinheit zur Verfügung gestellt. Dies kann sich insbesondere auf Kalibrationsdaten beziehen, die von der Sensoreinheit zur Bestimmung der Analyt-Konzentration benötigt werden.

Wichtig für die vorliegende Erfindung ist weiterhin, daß die Zentraleinheit des Sensor-Analysesystems zugleich das Auswertegerät des invasiven Element-Analysesystems ist und die Aufgabe der Kalibration des Sensor-Analyse-Teilsystems auf Basis von Meßdaten des Element-Analyse-Teilsystems übernimmt.

Das erfindungsgemäße Analysesystem zeichnet sich gegenüber den bisher praktisch gebräuchlichen Analysesystemen vor allem dadurch aus, daß ständig aktuelle Analysedaten verfügbar sind und zuverlässige Informationen über die Änderungsgeschwindigkeit der Glucosekonzentration zu jedem Zeitpunkt vorliegen. Dies ist besonders wichtig für Risikogruppen unter den Diabetikern, beispielsweise Diabetiker, die während der Nachtruhe zur Hypo- oder Hyperglykämie neigen. Auch unter erhöhter körperlicher Anstrengung (zum Beispiel beim Sport) kommt es in besonderem Maße auf die permanente Kontrolle der Glucosewerte an. Die Möglichkeit, den augenblicklichen Trend des Glucosewertes ("steigend" oder "fallend") qualitativ und quantitativ bestimmen zu können, ist für Diabetiker, die mit Insulin therapiert werden, zur Bestimmung der benötigten Insulinmenge von besonderer Bedeutung.

Die Erfindung wird im folgenden anhand von in dem Figuren schematisch dargestellten Ausführungsbeispielen näher erläutert. Es zeigen:
- Fig. 1: eine perspektivische Darstellung der Komponenten eines erfindungsgemäßen Analysesystems,
- Fig. 2: ein Blockdiagramm zur Erläuterung der Funktionen,
- Fig. 3: eine graphische Darstellung des zeitlichen Verlaufes von Analysedaten zur Erläuterung eines Kalibrationsverfahrens,
- Fig. 4: eine Aufsicht auf eine Zentraleinheit mit einer ersten Variante eines Graphik-Display,
- Fig. 5: eine Aufsicht auf eine Zentraleinheit mit einer zweiten Variante eines Graphik-Display.

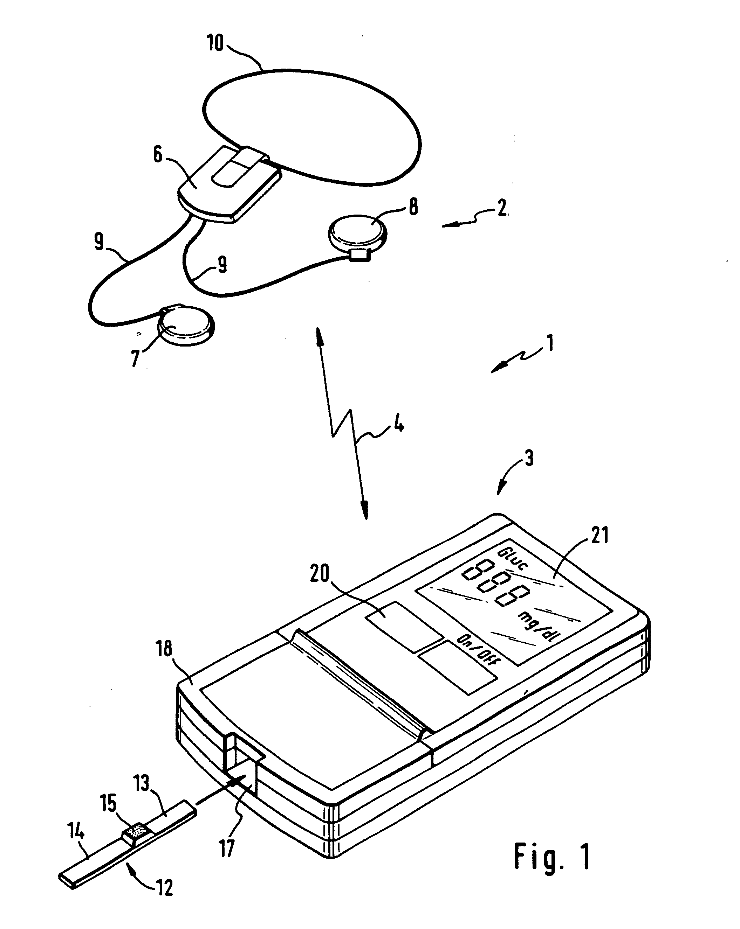

Das in Fig. 1 dargestellte integrierte Analyseelement-Sensor-Analysesystem (integrated analysis-element-sensor system, IASS) 1 besteht aus einem Analyseelement-Teilsystem und einem Sensor-Teilsystem. Eine am Körper des Patienten tragbare Sensoreinheit 2 ist mit einer Zentraleinheit 3 über eine durch den Pfeil 4 symbolisierte drahtlose Datenübertragung verbunden. Die Sensoreinheit 2 besteht in der dargestellten bevorzugten Ausführungsform aus einer Basiseinheit 6 und zwei Sensoren 7,8, die über Kabel 9 mit der Basiseinheit 6 verbunden sind. Die Basiseinheit 6 kann mit einem Halsband 10 um den Hals des Patienten getragen werden. Selbstverständlich kann sie auch in anderer Weise, beispielsweise mit Hilfe eines Schultergurtes oder eines Gürtel-Clips am Körper des Patienten befestigt sein.

Selbstverständlich kann grundsätzlich mit nur einem Sensor gearbeitet werden. Die Verwendung von zwei oder mehr Sensoren kann jedoch vorteilhaft sein, um gleichzeitig an mehreren Meßorten des Körpers einen mit der Glucosekonzentration korrelierenden Parameter messen zu können, wobei zur Erhöhung der Genauigkeit eine Mittelung der Meßwerte oder eine Auswahl des besseren Meßwertes anhand vorgegebener Zuverlässigkeits-Kriterien getroffen werden kann.

Die Zentraleinheit 3 weist die typischen Merkmale eines Auswertegerätes eines Element-Analysesystems auf. Im dargestellten Fall dient sie zur Auswertung eines Analyseelementes 12, welches als Glucose-Teststreifen 13 mit einer Basisschicht 14 und einem Testfeld 15 ausgebildet ist. Zur Auswertung wird das Analyseelement 12 in einen Meßschacht 17 eingeführt, der sich unter einer Klappe 18 des Gerätes 3 befindet. Zur Bedienung der Zentraleinheit 3 ist ein Tastenfeld 20 vorgesehen. Zur Ausgabe von Information, insbesondere zur Anzeige von Analysedaten dient ein Display 21.

Das in die Zentraleinheit 3 integrierte Analyseelement-Auswertegerät ist konventionell aufgebaut und muß deswegen nicht näher beschrieben werden. Nähere Informationen können aus zahlreichen Publikationen entnommen werden. Zum allgemeinen Geräteaufbau sei beispielsweise auf die europäische Patentanmeldung 0 492 326 und zu einer möglichen Meßelektronik auf die europäische Patentanmeldung 0 075 767 verwiesen.

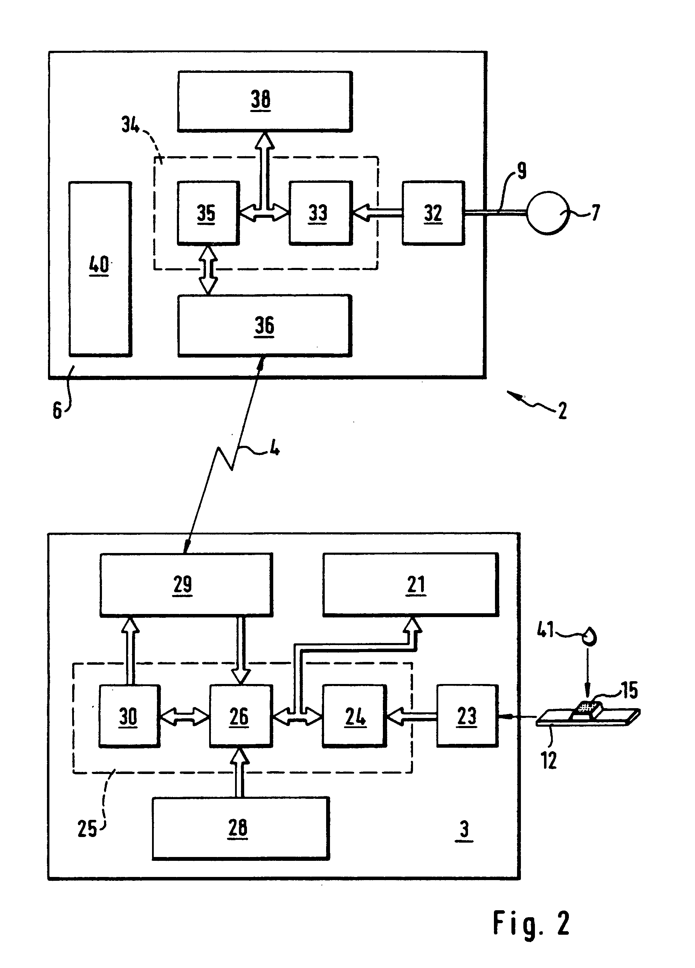

In Fig. 2 sind die wesentlichen Funktionskomponenten der Zentraleinheit 3 und der Sensoreinheit 2 als Blockdiagramm dargestellt.

Die Zentraleinheit 3 enthält eine Meßeinrichtung 23 zum Messen einer mit der Konzentration des Analyten korrelierenden Veränderung des Analyseelementes 12, beispielsweise ein Reflexionsphotometer, mit dem eine Farbänderung des Testfeldes 15 gemessen werden kann. Die Meßeinrichtung 23 erzeugt elektrische Signale, die dem gemessenen Wert der mit der Konzentration korrelierenden Veränderungen entsprechen und als Meßwert R bezeichnet werden.

Die gemessenen Meßwerte R werden an eine Auswerteelektronik 24 weitergeleitet, die Teil eines Mikrocomputers 25 ist, zu dem auch ein Datenspeicher 26 gehört. Die Auswerteelektronik 24 berechnet mit Hilfe einer in dem Speicher 26 gespeicherten Auswertekurve, die den funktionalen Zusammenhang der gesuchten Konzentration C von dem Meßwert R beschreibt (C_{A}=f(R)) die gesuchte Konzentration C des Analyten und gibt diese Analysedaten an den Speicher 26 weiter, wo sie als Element-Analysedaten C_{A} abgespeichert werden. Die Anzeige der Analysedaten C_{A} erfolgt - automatisch oder auf einen gesonderten Befehl - über das Display 21.

Die Auswertekurve (C_{A}=f(R)) kann in der Zentraleinheit 3 fest abgespeichert sein. Vorzugsweise wird jedoch für jede neue Herstellungscharge von Analyseelementen 12 eine spezielle chargenspezifische Auswertekurve verwendet, die der Zentraleinheit auf einem geeigneten Datenträger in maschinenlesbarer Form mitgeteilt wird. Zu diesem Zweck weist die Zentraleinheit einen Datenleser 28 auf, der beispielsweise einen Barcode-Leser sein kann, um einen auf den Analyseelementen selbst oder einem zusätzlichen Codeträger angebrachten Barcode zu lesen. Der Barcode ist jeder Analyseelement-Packung beigefügt und enthält die chargenspezifische Auswertekurve. Näheres hierzu ist beispielsweise in der europäischen Patentanmeldung 0 492 326 beschrieben.

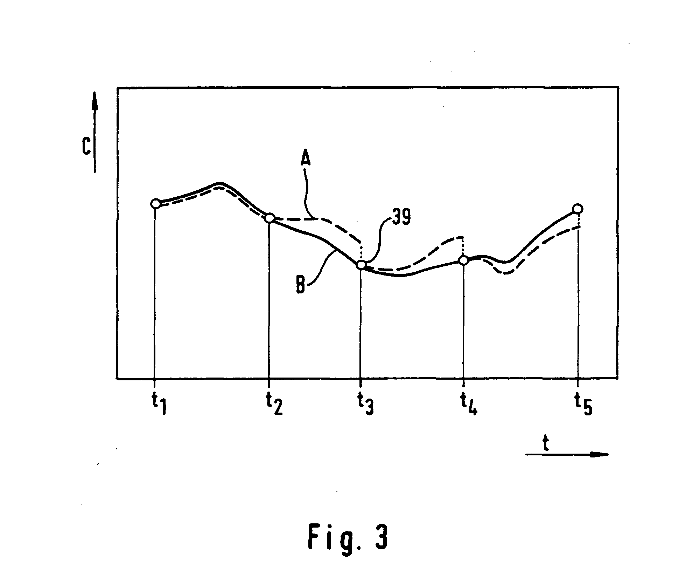

Zusätzlich zu den bisher beschriebenen Funktionsbausteinen, die bei Analyseelement-Auswertegeräten gebräuchlich sind, weist die Zentraleinheit 3 einen Sende- und Empfangsteil 29 zur drahtlosen Datenübertragung und Sensor-Kalibrationsmittel 30 auf, die in der Praxis vorzugsweise softwaremäßig realisiert und deswegen in Fig. 3 als Bestandteile des Mikrocomputers 25 eingezeichnet sind. Diese Komponenten verknüpfen das Analyseelement-Teilsystem mit dem Sensor-Teilsystem wie nachfolgend noch näher erläutert wird.

Die Basiseinheit 6 der Sensoreinheit 2 enthält eine Sensorbetriebseinheit 32, an die mindestens ein Sensor 7 angeschlossen ist. Die Sensorbetriebseinheit 32 enthält die Elemente, die erforderlich sind, um den Sensor 7 zu betreiben und dabei einen mit der Konzentration der Glucose korrelierenden Parameter an dem Körper des Patienten zu messen. In dem erwähnten bevorzugten Beispiel gehören hierzu Mittel zum Einstrahlen von Licht, wobei diese in dem Sensor 7 selbst angeordnete Leuchtdioden sein können, die über das Kabel 9 mit Strom versorgt werden. Alternativ können auch ein oder mehrere Lichtsender in der Basiseinheit 6 angeordnet sein, wobei das Kabel 9 Lichtleiter enthält, durch die das Licht in den Sensor 7 transportiert wird. In entsprechender Weise sind in dem Sensor 7 und/oder in der Basiseinheit 6 Lichtdetektionsmittel (z.B. Halbleiter-Lichtempfänger) vorgesehen, die das Licht nach Wechselwirkung mit dem Gewebe des Patienten detektieren. Weiter enthält die Sensorbetriebseinheit 32 elektronische Bauteile, wie beispielsweise Verstärker, um das empfangene Signal so aufzubereiten, daß ein Sensormeßwert S gewonnen wird, der mit der Analytkonzentration korreliert. Der Sensormeßwert S wird an Sensor-Auswertemittel 33 weitergeleitet, die vorzugsweise Bestandteile eines in die Basiseinheit 6 integrierten Mikrocomputer-Systems 34 sind, zu dem außerdem eine Speichereinheit 35 gehört. Die Sensor-Auswertemittel 33 berechnen - ebenso wie die Analyseelement-Auswertemittel 24 in der Praxis softwaremäßig - aus den Meßwerten S Analysedaten (Konzentrationen) C anhand einer Auswertekurve C_{S}=g(S), welche in der Speichereinheit 35 abgespeichert ist. Die Kalibrationskurve C_{S}=g(S)wird der Basiseinheit 6 von der Zentraleinheit 3 drahtlos übermittelt. Zu diesem Zweck weist die Basiseinheit 6 einen Sende- und Empfangsteil 36 auf, das die drahtlose Datenübertragung zwischen den beiden Einheiten 3,6 im Zusammenwirken mit dem Sende- und Empfangsteil 29 der Zentraleinheit ermöglicht.

Die berechneten Konzentrationsdaten (Sensor-Analysedaten C_{S}) werden in der Speichereinheit 35 abgespeichert. Sie können mittels einer Ausgabeeinheit 38 unabhängig von der Zentraleinheit 3 ausgegeben werden, wobei die an der Basiseinheit 6 vorgesehene Ausgabeeinheit 38 so gestaltet ist, daß sie möglichst klein ist und wenig Batterie verbraucht. Ihre Aufgabe ist in erster Linie eine Warnfunktion für den Fall, daß die Glucosekonzentration kritische Grenzwerte unter- oder überschreitet. Zweckmäßigerweise kann die Ausgabeeinheit 38 in Form einer Leuchtdiodenanzeige mit drei Leuchtdioden (für "Normalbereich", "Überzuckerungsgefahr", "Unterzuckerungsgefahr") realisiert sein. Alternativ oder zusätzlich kann eine akustische Signalausgabe vorgesehen sein.

In Fig. 2 eingezeichnet ist als Bestandteil der Basiseinheit 6 die Stromversorgungsbatterie 40. Dies ist wichtig, weil der Energieverbrauch von Analysesensoren verhältnismäßig hoch ist. Zweckmäßigerweise ist deswegen die Batterie 40 wiederaufladbar und in die Basiseinheit 6 ist eine nicht dargestellte Batteriespannungs-Überwachung integriert, die rechtzeitig auf das Erfordernis eines Batteriewechsels aufmerksam macht.

Bei der Benutzung des erfindungsgemäßen Analysesystems kann der Patient sich mit der Sensoreinheit 2 problemlos für längere Zeit von der stationären Zentraleinheit 3 entfernen. In dieser Zeit werden die Sensor-Analysedaten C_{S} in dem Speicher 35 gespeichert. Wenn der Patient nach Hause zurückkehrt und nahe genug bei der Zentraleinheit 3 ist, daß ein drahtloser Datenaustausch zwischen den Einheiten 3 und 6 möglich wird, werden die mittlerweile gewonnenen Sensor-Analysedaten C_{S} von dem Speicher 35 in den Speicher 26 der Zentraleinheit 3 transferiert. Der Patient kann dann jederzeit eine Kalibration mit Hilfe eines Analyseelementes 12 durchführen. Zweckmäßigerweise enthält die Zentraleinheit 3 eine Zeitmeßeinrichtung, die den Patient ausreichend häufig an die Durchführung einer Analyseelement-Analyse zur Kalibration erinnert. Jedesmal wenn eine solche Analyse durchgeführt wird, wird eine neue Auswertekurve C_{S}=g(S) in der Zentraleinheit 3 bestimmt und an die Sensoreinheit 2 übermittelt. Das Zusammenwirken der Einheiten 3 und 6 bei der Kalibration des Gesamtsystems wird nachfolgend erläutert.

Die Element-Analysedaten C_{A} werden wie beschrieben durch die Auswertekurve C_{A}=f(R) kalibriert, die vorzugsweise in Form eines maschinenlesbaren Codes über den Datenleser 28 in den Speicher 26 eingelesen wird. Demzufolge sind die Element-Analysedaten C_{A} mit guter Genauigkeit richtig.

Ein C_{A}-Wert liegt jeweils vor, wenn der Patient durch Stich in den Finger einen Blutstropfen 41 gewonnen und diesen mit Hilfe des Analyseelementes 12, der Meßeinrichtung 23 und der Auswerteelektronik 24 analysiert hat. Dies kann in größeren zeitlichen Abständen, beispielsweise ein- oder zweimal täglich geschehen. In Fig. 3 sind die zu Zeitpunkten t₁ bis t₅ bestimmten Analyseelement-Analysedaten als Punkte 39 eingezeichnet.

Der Sensor 7 erzeugt mit Hilfe seiner Sensorbetriebseinheit 32 und Auswerteelektronik 33 Sensor-Analysedaten C_{S}, wobei diese Messung kontinuierlich oder in so engen zeitlichen Abständen erfolgt, daß ein praktisch kontinuierlicher Verlauf von C_{S} in dem Speicher 35 abgespeichert und an die Zentraleinheit 3 übermittelt werden kann, wenn die Einheiten 2,3 in Datenübertragungs-Kontakt zueinander stehen. Fig.3 zeigt den zeitlichen Verlauf der C_{S}-Werte als gestrichelte Linie A. Zur Kalibration der Sensor-Analysedaten C_{S} werden Element-Analysedaten C_{A} verwendet. Dies kann beispielsweise in der Weise geschehen, daß jeweils zu den Kalibrationszeitpunkten t₁ bis t₅ die Sensor-Kalibrationsmittel 30 einen Vergleich der Analysedaten C_{A} und C_{S} durchführen, die in dem Speicher 26 abgespeichert sind. Aus diesem Vergleich ermitteln die Sensor-Kalibrationsmittel 30 eine neue korrigierte Auswertekurve C_{S}=g(S) und übermittelt diese über die Sende- und Empfangsteile 29,36 an die Basiseinheit 6, wo die neue Auswertekurve in dem Speicher 35 für künftige Berechnungen von Sensor-Analysedaten C_{S} mit Hilfe der Sensor-Auswerteelektronik 33 gespeichert wird. Zugleich kann die bei der Kalibration ermittelte neue Auswertekurve C_{S}=g(S) verwendet werden, um bereits in dem Speicher 26 abgespeicherte Sensor-Analysedaten rückwärts mindestens bis zu dem Zeitpunkt der vorhergehenden Element-Analyse nachzukorrigieren. Bei dem in Fig. 3 dargestellten Beispiel kann also aufgrund des zum Zeitpunkt t₂ gewonnenen Analyseelement-Konzentrationswertes C_{A} (t₂) eine Rückwärtskorrektur bis zu dem Zeitpunkt t₁ erfolgen. Entsprechendes gilt für den Zeitpunkt t₃ rückwärts bis t₂ usw. Der nach Kalibration korrigierte Verlauf der Sensor-Analysedaten ist in Fig. 3 als durchgezogene Linie B eingezeichnet.

Der Kalibrationsvorgang wurde vorstehend beispielhaft erläutert. Selbstverständlich kann die Kalibration in anderer Weise erfolgen, insbesondere in Anpassung an die Auswerteverfahren, mit denen die Analysedaten C_{S} bzw. C_{A} ermittelt werden. So eignen sich insbesondere zur Ermittlung von C_{S} numerische mathematische Verfahren, die aus einer Vielzahl von Meßdaten (beispielsweise Intensitätswerten bei vielen verschiedenen Wellenlängen) jeweils einen Konzentrationswert ermitteln. Erwähnt sei das PLS (partial least squares-Verfahren).

Die Speicherkapazität der Speicher 35 und 26 in der Sensoreinheit 2 bzw. der Zentraleinheit 3 ist den unterschiedlichen Einsatzzwecken angepaßt. Der Speicher 35 dient lediglich der mittelfristigen Speicherung verhältnismäßig kleiner Datenmengen, nämlich der Konzentrationswerte für den maximalen Zeitraum, für den sich der Patient mit seiner Sensoreinheit 2 von der Zentraleinheit 3 entfernt. Vorzugsweise ist die Speicherkapazität des Speichers 35 für die in einem Zeitraum von mindestens 2, vorzugsweise mindestens 8 Stunden anfallende Datenmenge ausgelegt. Der Speicher 26 der Zentraleinheit 3 ist im Regelfall erheblich größer und kann Analysedaten sowie Kalibrationsdaten, die über längere Zeiträume (mindestens etwa eine Woche) anfallen, aufnehmen. Zweckmäßigerweise ist die Zentraleinheit 3 mit einer nicht dargestellten Datenschnittstelle versehen, mit der diese Daten von Fall zu Fall zur weiteren Verarbeitung, beispielsweise an einen zur Speicherung der Patientendaten in der Arztpraxis verwendeten PC, übermittelt werden.

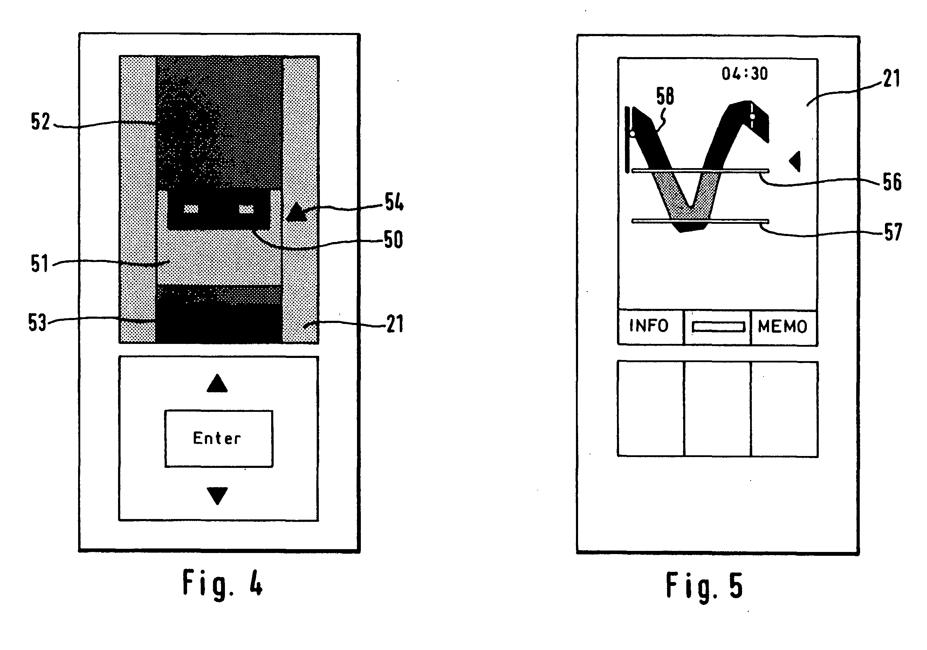

Die Anzeige der Analysedaten an der Zentraleinheit 3 kann alphanumerisch erfolgen, wie dies in Fig. 1 dargestellt ist. Vorzugsweise ist das Display 21 der Zentraleinheit 3 als Graphik-Display gestaltet, welches eine graphische Darstellung des zeitlichen Verlaufes der Sensor-Analysedaten ermöglicht. Beispielsweise wird bei der in Fig. 4 dargestellten Gestaltung des Display der augenblickliche Glucosewert durch den schwarzen Balken 50 in der Displaymitte symbolisiert. Das hellgraue Displayfeld 51 entspricht dem Normalbereich der Glucosewerte, während das dunkelgraue Feld 52 den oberen Warnbereich (Gefahr von Hyperglykämie) und das untere schwarze Feld 53 den unteren Warnbereich (Gefahr von Hypoglykämie) darstellt. Ein Pfeil in dem Display 54 zeigt den aktuellen Trend (hier zu steigenden Glucosewerten) an.

Bei der Graphik-Display-Darstellung von Fig. 5 wird der zeitliche Verlauf der Glucosewerte über einen längeren Zeitraum sichtbar. Der Normbereich der Glucosewerte ist durch zwei Warngrenzen 56,57 in der Displaymitte zu erkennen. Der Verlauf der Glucosewerte wird als relativ breiter Balken 58 dargestellt. In der Figur befindet er sich seit einiger Zeit im oberen Warnbereich und beginnt (beispielsweise infolge einer Insulininjektion) gerade zu fallen.

Die Graphik-Display-Darstellungen der Figuren 4 und 5 nutzen die besondere Fähigkeit des erfindungsgemäßen Systems, Glucosewerte praktisch kontinuierlich zuverlässig zu bestimmen. Die Auswerteeinrichtung 24 der Zentraleinheit 3 (unter Umständen auch die Auswerteeinrichtung 33 der Basiseinheit 6) enthält zu diesem Zweck (wiederum vorzugsweise softwaremäßig realisierte) Differenzierungsmittel, die es jederzeit ermöglichen, die zeitliche Ableitung des Verlaufs der Glucosewerte und damit den Trend zu bestimmen. Diese zusätzliche Information ist für die Therapie des Diabetes mellitus von erheblichem Wert.

## Patentansprüche

1. Analysesystem zur Überwachung der Konzentration eines Analyten im Blut eines Patienten, umfassend
ein Analyseelement-Teilsystem mit Analyseelementen (12), welche Reagenzien enthalten, deren Reaktion mit dem Analyten zu einer meßbaren mit der Konzentration des Analyten korrelierenden Veränderung (R) des Analyseelementes (12) führt, wenn man dieses mit einem Blutstropfen des Patienten kontaktiert und mit einem Auswertegerät, das eine Meßeinrichtung (23) zum Messen der Veränderung und Auswertemittel (24) zur Bestimmung von Element-Analysedaten CA aus den dabei erhaltenen Meßwerten (R) aufweist,
und ein Sensor-Teilsystem mit einer zur ständigen Gewinnung von Analysedaten am Körper des Patienten tragbaren Sensoreinheit (2), die einen Sensor (7) zur reagenzienfreien unmittelbaren Messung eines mit der Konzentration des Analyten korrelierenden Parameters an dem Körper des Patienten und zur Erzeugung von Sensormeßdaten (S), einen Sender (36) zum drahtlosen Senden von Datensignalen, sowie Sensor-Auswertemittel (33) zur Ermittlung von Sensor-Analysedaten C_{S} aus den Sensormeßdaten (S) einschließt,
wobei das Auswertegerät des Analyseelement-Teilsystems Teil einer Zentraleinheit (3) des Analysesystems ist, in der das Analyseelement-Teilsystem und das Sensor-Teilsystem dadurch verknüpft sind, daß sie
(i) einen Empfänger (29) und den Sender (36) zum drahtlosen Datenaustausch miteinander,
(ii) Kalibrationsmittel (30) zur Kalibration des Sensor-Teilsystems aufgrund der Element-Analysedaten C_{A} des Analyseelement-Teilsystems und
(iii) einen Datenspeicher (26) zur längerfristigen Speicherung von Analysedaten enthalten und
das Analysesystem so ausgebildet ist, daß der Patient eine Kalibration durchführen kann, wenn er mit dem an seinem Körper getragenen Sensor-Teilsystem nahe genug bei der Zentraleinheit (3) ist, so daß ein drahtloser Datenaustausch zwischen der Zentraleinheit (3) und dem Sensor-Teilsystem möglich ist.

2. Analysesystem nach Anspruch 1, **dadurch gekennzeichnet, daß** die Sensoreinheit (2) einen Lichtsender, von dem Licht in das Gewebe eines Körperteils des Patienten eingestrahlt wird und einen Lichtempfänger aufweist, durch den nach Wechselwirkung mit dem Gewebe aus dem Körperteil austretendes Licht detektiert wird, um eine durch die Wechselwirkung mit dem Gewebe veränderliche meßbare physikalische Eigenschaft des Lichts als mit der Konzentration des Analyten im Blut des Patienten korrelierenden Parameter zu bestimmen, aus dem die Sensor-Analysedaten C_{S} ermittelt werden.

3. Analysesystem nach Anspruch 1 oder 2, **dadurch gekennzeichnet, daß** die Sensoreinheit eine Basiseinheit (6) und einen mit der Basiseinheit (6) über Kabel (9) verbundenen Sensor (7) umfaßt, wobei die Basiseinheit (6) die Stromversorgungseinrichtung (40) der Sensoreinheit enthält.

4. Analysesystem nach Anspruch 3, **dadurch gekennzeichnet, daß** die Sensoreinheit (2) mindestens zwei Sensoren (7,8) aufweist, die über Kabel (9) mit einer gemeinsamen Basiseinheit (6) verbunden sind.

5. Analysesystem nach einem der vorhergehenden Ansprüche, **dadurch gekennzeichnet, daß** die Sensoreinheit (2) die Sensor-Auswertemittel (33) zur Ermittlung der Sensor-Analysedaten C_{S} einschließt.

6. Analysesystem nach Anspruch 5, **dadurch gekennzeichnet, daß** die Sensoreinheit (2) Anzeigemittel (38) für eine die Sensor-Analysedaten C_{S} repräsentierende Informationsausgabe aufweist.

7. Analysesystem nach Anspruch 5 oder 6, **dadurch gekennzeichnet, daß** sowohl der Sender (36) der Sensoreinheit (2) als auch der Empfänger (29) der Zentraleinheit (3) als Sender/Empfänger ausgebildet ist, um einen interaktiven Datenaustausch zwischen der Sensoreinheit (2) und der Zentraleinheit (3) zu ermöglichen.

8. Analysesystem nach einem der vorhergehenden Ansprüche, **dadurch gekennzeichnet, daß** die Sensoreinheit (2) einen Speicher (35) für die in einem Zeitraum von mindestens 2, vorzugsweise mindestens 8 Stunden ermittelten Sensor-Analysedaten C_{S} enthält.

9. Analysesystem nach einem der vorhergehenden Ansprüche, **dadurch gekennzeichnet, daß** die Zentraleinheit ein Graphik-Display (21) zur Darstellung des zeitlichen Verlaufs der Sensor-Analysedaten C_{S} aufweist.

10. Analysesystem nach Anspruch 9, **dadurch gekennzeichnet, daß** an dem Graphik-Display mehrere unterschiedliche Darstellungen (50-54;56-58) des zeitlichen Verlaufs der Sensor-Analysedaten C_{S} oder deren Änderungstendenz anzeigbar sind.

11. Verfahren zum Betrieb eines Systems nach einem der vorhergehenden Ansprüche, **dadurch gekennzeichnet, daß** jeweils zu dem Zeitpunkt, zu dem eine Analyseelement-Analyse durchgeführt wird, ein mit der Analyseelement-Analyse ermittelter Konzentrationswert C_{A} als Sollwert für die Kalibration der Sensor-Analysedaten C_{S} verwendet wird.

12. Verfahren nach Anspruch 11, **dadurch gekennzeichnet, daß** die Sensor-Analysedaten C_{S} aufgrund des Sollwertes mindestens bis zu dem Zeitpunkt der vorhergehenden Analyseelemente-Analyse automatisch nachkorrigiert. werden.

## Claims

1. An analytical system for monitoring the concentration of a substance to be analyzed in the blood of a patient, comprising
an element-analysis subsystem with analysis elements (12) containing reagents for reacting with the analyzed substance after the analysis element (12) has been brought in contact with a drop of blood of a patient thereby causing a measurable change (R) in the analysis-element (12) which correlates with the concentration of the analyzed substance, and with an evaluation instrument comprising a measurement device (23) to measure said change and evaluation means (24) to determine, from the measurement values (R) so obtained, element-analysis data C_{A},
and a sensor-analysis subsystem comprising a sensor unit (2) portable on the body of the patient for continuously obtaining analytical data, said sensor unit (2) including a sensor (7) borne on the patient body for the direct and reagent-free measurement of a parameter correlating with the concentration of the analyzed substance and for the generation of sensor measurement values (S), a transmitter (36) for the wireless transmission of data signals and sensor evaluation means (33) to determine sensor-analysis data C_{S} from the sensor measurement values (S), wherein
the evaluation instrument of the element-analysis subsystem is part of a central unit (3) of the analysis system, the element-analysis subsystem and the sensor-analysis subsystem being linked in that they comprise
(i) a receiver (29) and the transmitter (36) for wirelessly exchanging data signals;
(ii) calibration means (30) to calibrate the sensor-analysis subsystem on the basis of the element-analysis data C_{A} of the element-analysis subsystem and
(iii) a data memory (26) for the long-term storage of analytical data,
and wherein the analysis system is adapted to allow the patient to perform a calibration, when the patient and the sensor-analysis subsystem attached to the patient body is close enough to the central unit (3) to enable a wireless exchange of data signals between the central unit (3) and the sensor-analysis subsystem.

2. Analytical system according to claim 1, **characterized in that** the sensor unit (2) comprises a light source irradiating light into the tissue of a body part of the patient and a light detector detecting the light after it has interacted with the tissue of the body part in order to determine a physical light property which varies due to the interaction with the tissue thereby forming a parameter correlating with the concentration of the analyzed substance in the patient blood, the sensor-analysis data C_{S} being determined from said parameter.

3. Analytical system according to any one of claims 1 and 2, **characterized in that** the sensor unit comprises a base unit (6) and a sensor (7) connected by cables (9) to the base unit (6), the base unit (6) containing the power supply (40) for the sensor unit.

4. Analytical system according to claim 3, **characterized in that** the sensor unit (2) comprises at least two sensors (7, 8) connected by cables (9) to a common base unit (6).

5. Analytical system according to any one of the preceding claims, **characterized in that** the sensor unit (2) includes the sensor evaluation means (33) for determining the sensor-analysis data C_{S}.

6. Analytical system according to claim 5, **characterized in that** the sensor unit (2) comprises display means (38) for displaying information representing the sensor-analysis data C_{S}.

7. Analytical system according to any one of claims 5 or 6, **characterized in that** the transmitter (36) of the sensor unit (2) and the receiver (29) of the central unit (3) are adapted to form a transceiver system to allow interactive data exchange between the sensor unit (2) and the central unit (3).

8. Analytical system according to any one of the preceding claims, **characterized in that** the sensor unit (2) contains a memory (35) adapted for storing the sensor-analysis data C_{S} determined in a time interval of at least 2 hours, preferably at least 8 hours.

9. Analytical system according to any one of the preceding claims, **characterized in that** the central unit comprises a graph display (21) for the graphical representation of the time dependence of the sensor-analysis data C_{S}.

10. Analytical system according to claim 9, **characterized in that** a plurality of different display modes (50-54; 56-58) of the time dependence of the sensor-analysis data C_{S} or their variational trend can be displayed on the graph display.

11. A method for operating a system according to any one of the preceding claims, **characterized in that**, by the time an element-analysis is performed, a concentration value C_{A} determined in the element-analysis is used as the nominal value for the calibration of the sensor-analysis data C_{S}.

12. Method according to claim 11, **characterized in that** on the basis of the nominal value, the sensor-analysis data C_{S} are automatically back-corrected as far as the time of the previous element-analysis.

## Revendications

1. Système d'analyse pour surveiller la concentration d'un analyte dans le sang d'un patient, comprenant
un sous-système d'éléments d'analyse avec des éléments d'analyse (12) qui contiennent des réactifs dont la réaction avec l'analyte conduit à une modification (R) mesurable, en corrélation avec la concentration de l'analyte, de l'élément d'analyse (12) lorsqu'on met celui-ci en contact avec une goutte de sang du patient et avec un appareil d'évaluation doté d'un système de mesure (23) destiné à mesurer la modification et de moyens d'évaluation (24) destinés à déterminer des données d'analyse C_{A} de l'élément à partir des valeurs mesurées (R) obtenues,
et un sous-système de capteur avec une unité de capteur (2) pouvant être portée sur le corps du patient de manière à obtenir des données d'analyse de manière continue, laquelle comprend un capteur (7) destiné à mesurer immédiatement, sans réactifs, un paramètre en corrélation avec la concentration de l'analyte sur le corps du patient et à obtenir des données de mesure (S) du capteur, un émetteur (36) destiné à émettre sans fil des signaux de données ainsi que des moyens d'évaluation (33) du capteur destinés à déterminer des données d'analyse C_{S} du capteur à partir des données de mesure (S) du capteur,
l'appareil d'évaluation du sous-système d'éléments d'analyse faisant partie d'une unité centrale (3) du système d'analyse, dans laquelle le sous-système d'éléments d'analyse et le sous-système de capteur sont liés par le fait qu'ils contiennent
(i) un récepteur (29) et ledit émetteur (36) permettant l'échange de données sans fil entre eux,
(ii) des moyens de calibrage (30) permettant le calibrage du sous-système de capteur sur la base des données d'analyse C_{A} de l'élément du sous-système d'éléments d'analyse et
(iii) une mémoire de données (26) permattant le stockage à plus long terme de données d'analyse, et
le système d'analyse étant conçu de manière à ce que le patient puisse effectuer un calibrage s'il est suffisamment proche de l'unité centrale (3) avec le sous-système de capteur qu'il porte sur son corps, de façon à permettre un échange de données sans fil entre l'unité centrale (3) et le sous-système de capteur.

2. Système d'analyse selon la revendication 1, **caractérisé en ce que** l'unité de capteur (2) comprend un émetteur de lumière qui envoie de la lumière dans le tissu d'un élément du corps du patient, et un récepteur de lumière qui, après interaction avec le tissu, détecte la lumière sortant de l'élément du corps afin de déterminer une caractéristique physique mesurable et, du fait de l'interaction avec le tissu, variable de la lumière comme paramètre en corrélation avec la concentration de l'analyte dans le sang de patient, à partir duquel les données d'analyse Cs du capteur sont déterminées.

3. Système d'analyse selon la revendication 1 ou 2, **caractérisé en ce que** l'unité de capteur comprend une unité de base (6) et un capteur (7) relié à l'unité de base (6) par des câbles (9), l'unité de base (6) comprenant le dispositif d'alimentation (40) de l'unité de capteur.

4. Système d'analyse selon la revendication 3, **caractérisé en ce que** l'unité de capteur (2) comprend au moins deux capteurs (7, 8) qui sont reliés par des câbles (9) à une unité de base (6) commune.

5. Système d'analyse selon l'une des revendications précédentes, **caractérisé en ce que** l'unité de capteur (2) contient les moyens d'évaluation (33) du capteur destinés à déterminer les données d'analyse C_{S} du capteur.

6. Système d'analyse selon la revendication 5, **caractérisé en ce que** l'unité de capteur (2) comprend des moyens d'affichage (38) pour une sortie d'informations représentant les données d'analyse Cs du capteur.

7. Système d'analyse selon la revendication 5 ou 6, **caractérisé en ce qu'**à la fois l'émetteur (36) de l'unité de capteur (2) et le récepteur (29) de l'unité centrale (3) sont conçus comme émetteur-récepteur afin de permettre un échange de données interactif entre l'unité de capteur (2) et l'unité centrale (3).

8. Système d'analyse selon l'une des revendications précédentes, **caractérisé en ce que** l'unité de capteur (2) comprend une mémoire (35) pour les données d'analyse Cs du capteur déterminées sur une durée d'au moins 2, de préférence d'au moins 8 heures.

9. Système d'analyse selon l'une des revendications précédentes, **caractérisé en ce que** l'unité centrale comprend un écran graphique (21) pour présenter la variation temporelle des données d'analyse Cs du capteur.

10. Système d'analyse selon la revendication 9, **caractérisé en ce que** plusieurs présentations différentes (50-54 ; 56-58) de la variation temporelle des données d'analyse C_{S} du capteur ou de leur tendance à la modification peuvent être affichées sur l'écran graphique.

11. Procédé pour le fonctionnement d'un système selon l'une des revendications précédentes, **caractérisé en ce qu'**à chaque instant auquel une analyse de l'élément d'analyse est effectuée, une valeur de concentration C_{A} déterminée à l'aide de l'analyse de l'élément d'analyse est utilisée comme valeur de consigne pour le calibrage des données d'analyse C_{S} du capteur.

12. Procédé selon la revendication 11, **caractérisé en ce que** les données d'analyse C_{S} du capteur sont automatiquement recorrigées sur la base de la valeur de consigne, au moins jusqu'à l'instant de l'analyse précédente de l'élément d'analyse.
